(19) Europäisches Patentamt
European Patent Office
Office européen des brevets

(11) EP 0 703 971 B1

(12) **EUROPÄISCHE PATENTSCHRIFT**

(45) Veröffentlichungstag und Bekanntmachung des Hinweises auf die Patenterteilung:
**11.10.2000 Patentblatt 2000/41**

(21) Anmeldenummer: **94920434.1**

(22) Anmeldetag: **07.06.1994**

(51) Int. Cl.[7]: **C11D 3/33**, C07C 229/16, C07C 229/26, C07C 229/36, C07C 229/34, C07C 227/00, C07C 255/25

(86) Internationale Anmeldenummer:
**PCT/EP94/01838**

(87) Internationale Veröffentlichungsnummer:
**WO 94/29421 (22.12.1994 Gazette 1994/28)**

(54) **VERWENDUNG VON ALPHA-ALANIN-N,N-DIESSIGSÄURE UND IHREN SALZEN ALS BIOLOGISCH ABBAUBARE KOMPLEXBILDNER FÜR ERDALKALI- UND SCHWERMETALLIONEN IN REINIGUNGSFORMULIERUNGEN FÜR DIE GESCHIRREINIGUNG**

USE OF N,N-DIACETIC ALPHA-ALANINE AND ITS SALTS AS BIODEGRADABLE COMPLEXING AGENTS FOR ALKALINE EARTH AND HEAVY METAL IONS IN CLEANING COMPOSITIONS FOR DISHWASHING

UTILISATION D'ACIDE ALPHA ALANINE N,N-DIACETIQUE ET SES SELS COMME AGENT COMPLEXANTS BIODEGRADABLE POUR DES IONS DE METAUX LOURDS ET ALCALINO-TERREUX DANS DES COMPOSITIONS DE NETTOYAGE DE LA VAISELLE

(84) Benannte Vertragsstaaten:
**AT BE CH DE DK ES FR GB IT LI NL SE**

(30) Priorität: **16.06.1993 DE 4319935**

(43) Veröffentlichungstag der Anmeldung:
**03.04.1996 Patentblatt 1996/14**

(60) Teilanmeldung:
**99111836.5 / 0 976 818**
**97122735.0 / 0 846 753**
**97101598.7 / 0 781 762**

(73) Patentinhaber:
**BASF AKTIENGESELLSCHAFT**
**67056 Ludwigshafen (DE)**

(72) Erfinder:
- **SCHNEIDER, Juergen**
  **D-67251 Freinsheim (DE)**
- **POITHOFF-KARL, Birgit**
  **D-67061 Ludwigshafen (DE)**
- **KUD, Alexander**
  **D-55234 Eppelsheim (DE)**
- **BAUR, Richard**
  **D-67112 Mutterstadt (DE)**
- **OFTRING, Alfred**
  **D-67098 Bad Duerkheim (DE)**
- **GREINDL, Thomas**
  **D-94127 Neuburg (DE)**

(56) Entgegenhaltungen:
**DE-A- 2 027 972** **GB-A- 2 178 754**
**US-A- 2 500 019**

- **CHEMICAL ABSTRACTS, Band 58, Nr. 5, veröffentlicht 1963, 04 März, (Columbus, Ohio, USA), N.F.KAZARINOVA et al. "Investigation of complexing agents derived from amino acids", Nr. 4644e; Verbindungen V,IV.**
- **CHEMICAL ABSTRACTS, Band 95, Nr. 18, veröffentlicht 1981 02 November, (Columbus, Ohio, USA), T.NOGUCHI et al."Phosphorus- free detergent composition", Seite 100, Nr. 152 502q; & EP,A,0 030 461**
- **CHEMICAL ABSTRACTS, Band 63, Nr. 5, veröffentlicht 1965, 30 August, (Columbus, Ohio, USA), CHAS.PFIZER & CO."2-(N,N-BIS(carboxymethyl))amino-2- deoxy-D-glycero-D-gulo-heptonic acid", Nr. 5732a; & GB,A,989 926.**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 703 971 B1



(Forts. nächste Seite)

- **CHEMICAL ABSTRACTS, Band 103, Nr. 14, veröffentlicht 1985, 07 Oktober, (Columbus, Ohio, USA), S.LUBKEOVA et al."New complexanes.VL. Acid-base and chelate-forming properties of surface-active complexanes of the type of 2-alkylnitrilotriacetic acids", Seite 396, Nr. 110 849m; Chem.Pap. 1985, 39(3), 317-327.**
- **CHEMICAL ABSTRACTS, Band 106, Nr. 18, veröffentlicht 1987, 04 Mai, (Columbus, Ohio, USA), S.LUBKEOVA et al."Surfactants from monoamine complexons", Seite 103, Nr. 140 140c; & CZ,A,199 072.**

**Beschreibung**

Die vorliegende Erfindung betrifft die Verwendung von α-Alanin-N,N-diessigsäure und ihren Alkalimetall-, Erdalkalimetall-, Ammonium- und substituierten Ammoniumsalzen als Komplexbildner für Erdalkali- und Schwermetallionen in Reinigungsmittelformulierungen für die Geschirreinigung.

Aus den japanischen Offenlegungsschriften 80/157 695 (1) und 80/160 099 (2), zitiert in Chem. Abstr. 95 (1981), 9123 m bzw. 9124 n, ist bekannt, Alanin-N,N-diessigsäure in Form des Natriumsalzes als Builder in pulverförmig formulierten Textilwaschmitteln einzusetzen, wobei insbesondere für Baumwolltextilien eine Waschkraftverstärkung zu beobachten ist.

Die EP-A 089 136 (3) betrifft Mundhygieneprodukte, die als Calciumsequestriermittel u.a. α-Alanin-N,N-diessigsäure enthalten. Diese dienen dazu, die Menge an Calciumfluorid, welche dem Zahnschmelz zum Kariesschutz zugeführt wird, zu kontrollieren.

Aus der Literaturstelle Tr. Soveshch. po Kompleksonam, Akad. Nauk SSSR, Ural'sk. Filial, Sverdlovsk 1958, 39-52 (zitiert in Chem. Abstr. 58, 4644e, 1963) ist bekannt, daß sich α-Alanin-N-diessigsäure als Komplexbildner für Schwermetallionen eignet. Hinweise auf mögliche Anwendungen werden nicht gegeben.

Als Komplexbildner für Erdalkali- und Schwerinetallionen auf den verschiedensten technischen Gebieten mit ihren teilweise stark voneinander abweichenden Anforderungs- und Problemfeldern werden üblicherweise immer noch altbekannte und bewährte Systeme wie Polyphosphate, Nitrilotriessigsäure oder Ethylendiamintetraessigsäure eingesetzt. Diese Mittel zeigen allerdings gewisse Nachteile, prinzipielle Schwachpunkte sind insbesondere ihr noch verbesserungsbedürftiges Calcium- und Mangan-Bindevermögen, ihre noch nicht optimale stabilisierende Wirkung in Bleichbädern und Bleichsystemen sowie ihre meist unzureichende biologische Abbaubarkeit bzw. Eliminierbarkeit.

Aufgabe der vorliegenden Erfindung war es daher, Komplexbildner bereitzustellen, welche die Nachteile des Standes der Technik nicht mehr aufweisen.

Demgemäß wurde die Verwendung von α-Alanin-N,N-diessigsäure und ihren Salzen der allgemeinen Formel I

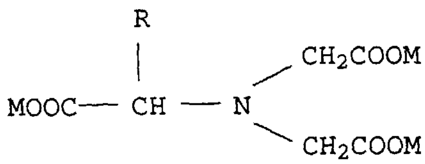

(I)

in der

R für Methyl steht und

M Wasserstoff, Alkalimetall, Ammonium oder substituiertes Ammonium in den entsprechenden stöchiometrischen Mengen bedeutet,

als Komplexbildner für Erdalkali- und Schwermetallionen in Reinigungsmittelformulierungen für die Geschirreinigung gefunden.

Als derartige Salze eignen sich vor allem die Natrium-, Kalium- und Ammoniumsalze, insbesondere das Trinatrium-, Trikalium- und Triammoniumsalz sowie organische Triaminsalze mit einem tertiären Stickstoffatom.

Als den organischen Aminsalzen zugrundeliegende Basen kommen insbesondere tertiäre Amine, wie Trialkylamine mit 1 bis 4 C-Atomen im Alkyl, wie Trimethyl- und Triethylamin, und Trialkanolamine mit 2 oder 3 C-Atomen im Alkanolrest, bevorzugt Triethanolamin, Tri-n-propanolamin oder Triisopropanolamin, in Betracht.

Die erfindungsgemäße Verwendung für α-Alanin-N,N-diessigsäure und ihre Salze I liegt in Geschirreinigungsmittelformulierungen, insbesondere in phosphatfreien Mitteln für das maschinelle Geschirreinigen in Geschirrspülmaschinen im Haushalt oder in Gewerbebetrieben, z. B. Großküchen oder Restaurants.

Eine vorteilhafte Wirkung der α-Alanin-N,N-diessigsäure bzw. ihrer Salze I liegt in der Bleichmittelstabilisierung. Spuren von Schwermetallen wie Eisen, Kupfer und Mangan kommen in den Komponenten der Geschirreinigungsmittelformulierung selbst, im Wasser und im zu bleichenden Gut vor und katalysieren die Zersetzung des Bleichmittels. Die Komplexbildner I binden diese Metallionen und verhindern die unerwünschte Zersetzung des Bleichsystems während der Lagerung und bei der Anwendung. Dadurch erhöht sich die Effizienz des Bleichsystems.

α-Alanin-N,N-diessigsäure und ihre Salze I eignen sich vor allem deshalb so gut für den beschriebenen Anwendungszweck, weil sie außerordentlich effektive Komplexbildner für Erdalkalimetallionen und für Schwermetallionen, insbesondere für Calcium und Mangan, darstellen. Ihr Calcium- und ihr Mangan-Bindevermögen sind außergewöhnlich hoch.

Weitere Vorteile sind ihr sehr geringes Toxizitätspotential und ihre gute biologische Abbaubarkeit. So zeigt α-Alanin-N,N-diessigsäure im Zahn-Wellens-Test unter Standardbedingungen eine biologische Abbaubarkeit > 90 % (28-Tage-Wert), wogegen beispielsweise Ethylendiamintetraessigsäure unter gleichen Bedingungen einen Wert von < 10 % ergibt.

Gegenstand der vorliegenden Erfindung sind auch Geschirreinigungsmittelformulierungen, insbesondere solche für das maschinelle Geschirreinigen in Geschirrspülmaschinen im Haushalt und in Gewerbebetrieben, welche wirksame Mengen an α-Alanin-N,N-diessigsäure oder ihren Salzen I enthalten.

Herstellungsbeispiele

Beispiel 1

Herstellung von α-D,L-Alanin-N,N-diessigsäure-Trinatriumsalz aus Iminodiacetonitril

Zu einer Suspension von 95 g Iminodiacetonitril (100 gew.-%ig) in 500 ml Wasser wurden nacheinander bei 35 bis 50°C 14 g Schwefelsäure (100 gew.-%ig), 27 g wasserfreie Blausäure und 44 g Acetaldehyd (100 gew.-%ig) gegeben. Man rührte solange, bis bei der Titration des Blausäuregehaltes keine Änderung mehr festzustellen war. Nach Abkühlen auf 10°C wurde der Niederschlag abfiltriert und getrocknet. Es resultierten 123,4 g α-D,L-Alaninnitril-N,N-diacetonitril (entsprechend 83 % der Theorie) vom Schmelzpunkt 82°C.

Das erhaltene α-D,L-Alaninnitril-N,N-diacetonitril wurde bei 50°C in 440 g 25 gew.-%iger wäßriger Natronlauge eingetragen, es wurde anschließend noch 2 Stunden bei dieser Temperatur nachgerührt.

Danach wurde für 10 Stunden auf 95°C erwärmt. Gegen Ende der Umsetzung wurde das Reaktionsgemisch mit Wasser verdünnt. Man erhielt so 610 g einer wäßrigen Lösung von α-D,L-Alanin-N,N-diessigsäure-Trinatriumsalz mit einem Eisen-Bindevermögen von 1,285 mmol/g (entsprechend 94 % der Theorie, bez. auf eingesetztes α-D,L-Alaninnitril-N,N-diacetonitril).

Beispiel 2

Herstellung von α-D-L-Alanin-N,N-diessigsäure-Trinatriumsalz aus α-D,L-Alanin

Zu einer Suspension von 44 g D,L-Alanin (> 99 gew.-%ig) in 200 g Wasser gab man bei 30°C gleichzeitig 105 g Formaldehyd (30 gew.-%ig) und 31,7 g Blausäure (89,5 gew.-%ig). Es wurde noch 3 Stunden bei 30°C nachgerührt. Die Blausäure-Ahnahme entsprach einem Umsatz von > 97 % der Theorie.

Die so erhaltene wäßrige Lösung von α-D,L-Alanin-N,N-diacetonitril wurde bei 30°C zu 132 g 50 gew.-%iger Natronlauge getropft. Nach 8-stündigem Rühren bei 30°C wurde die Temperatur auf 95 bis 102°C erhöht. Nach weiteren 4 Stunden war die Umsetzung praktisch vollständig. Man erhielt 352,5 g einer Lösung, die gemäß ihrem Eisen-Bindevermögen 37,4 Gew.-% α-D,L-Alanin-N,N-diessigsäure-Trinatriumsalz enthielt (entsprechend einer Ausbeute von 97,4 % der Theorie über beide Stufen).

Anwendungstechnische Daten und Anwendungsbeispiele

Bestimmung des Calcium-Bindevermögens

Meßprinzip

Die inhibierende Wirkung von Komplexbildnern oder Dispergiermitteln auf die Ausfällung von Calciumcarbonat wird durch Trübungstitration bestimmt. Es wird die zu untersuchende Substanz vorgelegt und in Gegenwart von Natriumcarbonat mit Calciumacetatlösung titriert. Der Endpunkt wird durch Bildung des Calciumcarbonat-Niederschlages angezeigt. Durch Verwendung einer ausreichenden Menge an Natriumcarbonat wird sichergestellt, daß die Messung auch dann ein korrektes Ergebnisliefert, wenn die Wirkung nicht nur auf einer Komplexierung der Calciumionen beruht, sondern auf der Dispergierung von Calciumcarbonat. Werden nämlich zu kleine Natriuncarbonatmengen eingesetzt, besteht die Gefahr, daß das Dispergiervermögen des Produktes nicht ausgeschöpft wird; in diesem Fall wird der Titrationsendpunkt durch die Fällung des Calcium-Salzes der untersuchten Verbindung bestimmt.

Während der Titration wird die Änderung der Lichtdurchlässigkeit mit Hilfe eines Lichtleiterphotometers verfolgt. Bei letzterem wird ein über Glasfaser in die Lösung geleiteter Lichtstrahl an einem Spiegel reflektiert und die Intensität des reflektierenden Lichts gemessen.

Reagenzien

0,25 m $Ca(OAc)_2$-Lösung
10 gew.-%ige $Na_2CO_3$-Lösung
1 n NaOH-Lösung
1 gew.-%ige Salzsäure

Durchführung

1 g Wirksubstanz (W.S.) in Form des Trinatriumsalzes wird in 100 ml destilliertem $H_2O$ gelöst. Anschließend werden 10 ml 10 gew.%ige $Na_2CO_3$-Lösung zugegeben. Bei Raumtemperatur (RT) und einem während der Titration konstant gehaltenen pH-Wert von 11 und bei 80°C mit einem pH-Wert von 10 wird mit 0,25 m $Ca(OAc)_2$-Lösung kontinuierlich mit 0,2 ml/min automatisch titriert.

Berechnung

Menge mg $CaCO_3$/g W.S. = Verbrauch an $Ca(OAc)_2$-Lösung in ml x 25. Bei der automatischen Titration ist der 1. Knickpunkt der Titrationskurve der Endpunkt.
Die folgende Tabelle 1 zeigt die Ergebnisse der Bestimmungen.

Tabelle 1

| Komplexbildner | Calciumcarbonat-Dispergier-Kapazität | | | |
|---|---|---|---|---|
| | mg $CaCO_3$/g RT pH 11 | W.S. 80°C pH 10 | $CaCO_3$/mol RT pH 11 | W.S. 80°C pH 10 |
| α-ADA-$Na_3$ aus Bsp. Nr. 2 | 370 | 330 | 1,00 | 0,89 |
| zum Vergleich: | | | | |
| Pentanatriumtriphosphat | 215 | 150 | 0,79 | 0,55 |
| NTA-$Na_3$ | 350 | 250 | 0,90 | 0,64 |
| EDTA-$Na_4$ | 275 | 240 | 1,04 | 0,91 |
| α-ADA-$Na_3$ = α-Alanin-N,N-diessigsäure-Trinatriumsalz<br>NTA-$Na_3$ = Nitrilotriessigsäure-Trinatriumsalz<br>EDTA-$Na_4$ = Ethylendiamintetraessigsäure-Tetranatriumsalz | | | | |

Bestimmung des Mangan-Bindevermögens

Meßvorschrift

10,0 ml 0,005 m $MnSO_4 \cdot H_2O$-Lösung werden mit 50 ml destilliertem Wasser, 10 Tropfen 5 gew.-%iger Kaliumnatriumtartratlösung, ca. 3 ml einer Pufferlösung, ca. 30 mg Ascorbinsäure und einer Spatelspitze Indikator (1 Gew.-Teil Eriochromschwarz T mit 400 Gew.-Teilen NaCl verrieben) versetzt und auf 75°C erwärmt. Diese Lösung wird mit einer 0,001 in Lösung des Komplexbildners (K.B.) bis zum bleibenden Umschlag nach blau titriert.

Auswertung

$$\text{mg Mn}^{2+}/\text{g K.B.} = \frac{274{,}7 \times 1000}{\text{verbrauchte ml} \times \text{Molgewicht K.B.}}$$

$$\text{mol Mn}^{2+}/\text{mol K.B.} = \frac{274{,}7 \times 1000}{\text{verbrauchte ml} \times 54{,}94}$$

Die folgende Tabelle 2 zeigt die Ergebnisse der Bestimmungen.

Tabelle 2

| Komplexbildner | mg $Mn^{2+}$/g Komplexbildner | mol $Mn^{2+}$/mol Komplexbildner |
|---|---|---|
| α-ADA-$Na_3$ aus Bsp. Nr. 2 | 209 | 0,86 |
| zum Vergleich: EDTA-$Na_4$ | 192 | 1,02 |

**Patentansprüche**

**1.** Verwendung von α-Alanin-N,N-diessigsäure und ihren Salzen der Formel I

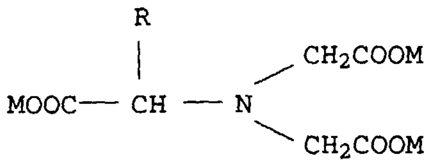

(I)

in der

R für Methyl steht und

M Wasserstoff, Alkalimetall, Ammonium oder substituiertes Ammonium in den entsprechenden stöchiometrischen Mengen bedeutet,

als Komplexbildner für Erdalkali- und Schwermetallionen in Reinigungsmittelformulierungen für die Geschirreinigung.

**2.** Geschirreinigungsmittelformulierungen, enthaltend wirksame Mengen an α-Alanin-N,N-diessigsäure oder ihren Salzen I gemäß Anspruch 1.

**3.** Geschirreinigungsmittelformulierungen nach Anspruch 2 für das maschinelle Geschirreinigen in Geschirrspülmaschinen im Haushalt oder in Gewerbebetrieben, enthaltend wirksame Mengen an α-Alanin-N,N-diessigsäure oder ihren Salzen I.

**Claims**

**1.** The use of α-alanine-N,N-diacetic acid and its salts of the formula I

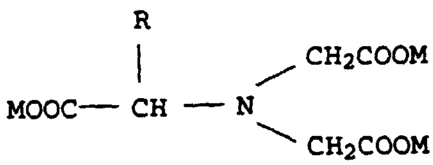

(I)

in which

R is methyl

M is hydrogen, alkali metal, ammonium or substituted ammonium in the appropriate stoichiometric amounts,

as complexing agents for alkaline earth metal ions and heavy metal ions in cleaner formulations for dishwashing.

**2.** A dishwashing composition formulation containing an effective amount of α-alanine-N,N-diacetic acid or its salts as claimed in claim 1.

**3.** A dishwashing composition formulation as claimed in claim 2 for mechanical dishwashing in dishwashing machines in the household or in commercial operations, containing an effective amount of α-alanine-N,N-diacetic acid or its salts I.

## Revendications

**1.** Utilisation d'acide N,N-diacétique d'α-alanine et de ses sels de formule I

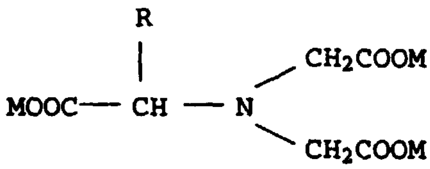

(I)

dans laquelle

R représente un groupement méthyle et
M représente un atome d'hydrogène, un atome de métal alcalin, un groupement ammonium ou ammonium substitué dans les quantités stoechiométriques correspondantes,

en tant qu'agent complexant pour des ions de métaux alcalino-terreux et de métaux lourds dans des formulations d'agents de nettoyage pour le nettoyage de la vaisselle.

**2.** Formulations d'agents de nettoyage pour la vaisselle contenant des quantités efficaces d'acide N,N-diacétique d'α-alanine ou de ses sels I selon la revendication 1.

**3.** Formulations d'agents de nettoyage pour la vaisselle selon la revendication 2 pour le nettoyage de la vaisselle en machine dans des lave-vaisselles dans des installations domestiques ou industrielles, contenant des quantités efficaces d'acide N,N-diacétique d'α-alanine ou de ses sels I.